# EUROPEAN PATENT SPECIFICATION

(11) **EP 0 476 021 B1**
(45) Date of publication and mention of the grant of the patent: **10.01.1996**
(21) Application number: 90909426.0
(22) Date of filing: 07.06.1990
(51) Int. Cl.: A61L 2/18

(54) **APPARATUS FOR STERILIZING CONTACT LENSES**
STERILISIERUNGSVORRICHTUNG FÜR KONTAKTLINSEN
APPAREIL DE STERILISATION DE LENTILLES DE CONTACT

(30) Priority: 09.06.1989 US 364471
(43) Date of publication of application: 25.03.1992
(73) Proprietor: CIBA VISION CORPORATION, Atlanta, GA 30360 (US)
(72) Inventor: CEROLA, Joseph, J., Guntersville, AL 35976 (US); PERLAKY, Steven, C., Arab, AL 35016 (US)
(74) Representative: Long, Edward Anthony
(86) International application number: US9003245
(87) International publication number: WO9014848

(56) References cited:
- US-A- 3 912 451
- US-A- 3 939 968
- US-A- 4 807 750
- US-A- 4 817 998

## Description

### Background of the Invention

This invention relates to a chemical sterilization process for articles such as contact lenses. More particularly, the invention concerns an improved apparatus adapted to be employed in said chemical sterilization process.

The sterilization of contact lenses is currently done by a number of different processes. Some processes employ heat, either in a dry sterilization environment, or with a sterilized saline or similar solution. A further process has also been developed which employs an anti-microbial sterilizing solution such as hydrogen peroxide (H₂O₂).

The present invention concerns an apparatus specifically designed for use with the latter (hydrogen peroxide) type of sterilization technique. One particularly useful method and apparatus is disclosed in prior patent 4,013,410, also assigned to the assignee of this application, to which reference is invited for background material concerning this type of sterilizing process.

Briefly, the hydrogen peroxide solution which is employed is of a relatively weak concentration, for example, no more than on the order of 3% solution of hydrogen peroxide in water. In this sterilizing process it is important to insure that the hydrogen peroxide solution, or any remnants thereof, are completely absent from the lenses before the lenses are reinserted by the wearer. Accordingly, the heretofore applied processes operate to neutralize the hydrogen peroxide solution well before the lenses are removed from the apparatus and replaced in the eye of the wearer.

Generally speaking, the process employed by the above referenced patent uses a catalytic agent such as platinum, which will produce a chemical reaction resulting in neutralization or decay of the hydrogen peroxide. Essentially, a free oxygen molecule is liberated and the hydrogen peroxide is reduced to water. Preferably, the process is arranged to take place over a period of several hours of time, depending on the nature of the catalytic agent and the initial strength of the original sterilizing solution. Accordingly, it is generally recommended that the user employ the process overnight in order to assure full decomposition of the hydrogen peroxide and a considerable period of soaking of the lenses in the sterile water which remains of the solution after decomposition of the hydrogen peroxide.

While the apparatus employed in the above-referenced patent has met with commercial success, there is nonetheless room for further improvement. More particularly, one apparatus currently in use employs a plastic carrier member which is coated with a suitable platinum catalytic agent, and which is generally disposed in the bottom of a cylindrical container. The container is then filled with the hydrogen peroxide solution and the contact lenses, which are held in a further lens carrier/lens support case member, are then disposed within the container. Generally speaking, the lens support or lens carrier members are well-ventilated basket-like structures which are affixed to a cap member which in turn forms a cap or top closure for the solution-filled container. It is important, however, to provide some venting of the cap portion of the closure to control pressure buildup by permitting the escape of some of the free oxygen generated during the decomposition of the hydrogen peroxide solution.

While this system has proven highly successful in operation, there is, as mentioned above, room for improvement. For example, it is necessary for the user to periodically handle the "catalyst" which has the coating of catalyst material. That is, the catalyst material is consumed over a period of use, and therefore the catalyst member must be removed and replaced with a new catalyst from time to time. Handling by the consumer has a number of drawbacks. Some of the platinun coating of the carrier may rub off onto the fingers and cause undesirable staining of the skin and/or clothing of the user. Moreover, various proteins, salts, etc., from the skin might compromise the integrity and action of the catalyst. It appears that to some extent sulfur compounds and the proteins in the skin may combine with the platinum to form an inert compound. Moreover, with the catalyst member disposed in the bottom of the container, it is somewhat difficult to reach it manually without the use of some sort of tool, or the like.

There is also some concern that with the catalyst sitting in the bottom of the container between uses and after rinsing the container following a use, that some excess water may be trapped or entrained around the catalyst member which may give some potential for bacterial growth.

### Objects and Summary of the Invention

It is an object of the invention to provide an improved apparatus for use in the foregoing lens sterilization process which further improves upon some of the prior art apparatus discussed hereinabove.

Briefly, and in accordance with the foregoing discussion, the invention provides apparatus for use in the sterilization of contact lenses or the like, wherein said lenses are disposed within a sterilizing solution contained within a reaction vessel, which sterilizing solution is capable of being decomposed through contact with a catalytic agent. The apparatus in accordance with the invention comprises a reaction vessel capable of containing a sterilizing solution and comprising a container portion, cover means and means for supporting an article in contact with sterilizing solution in said container; and further including means for mounting a catalytic agent on said supporting means such that said catalytic agent is removable for replacement by a user as the catalytic agent becomes exhausted in use; said mounting of said catalytic agent on said supporting means thereby preventing contact of the catalytic agent with the solution in said container until the article supporting means with the contact lenses therein are placed in the solution in the container for beginning the sterilizing process, to thereby prevent premature breakdown of the sterilizing solution prior to the introduction of contact lenses for sterilization therein.

### Brief Description of the Drawings

The features of the present invention which are believed to be novel are set forth with particularity in the appended claims. The organization and manner of the operation of the invention, together with further objects and advantages thereof may best be understood by reference to the following description, taken in connection with the accompanying drawings in which like reference numerals identify like elements, and in which:
Fig. 1 is a partially exploded perspective view of apparatus in accordance with the invention;
Fig. 2 is an enlarged sectional view of a fully assembled apparatus of the invention in operation for sterilizing contact lenses;
Fig. 3 is a reduced sectional view taken generally along the line 3-3 of Fig. 2;
Fig. 4 is an enlarged exploded perspective view illustrating a frame-like carrier member and a disc-like catalyst member in accordance with a preferred form of the invention;
Fig. 5 is a transverse section taken on the assembled carrier and catalyst members of Fig. 4;
Fig. 6 is an enlarged partial sectional view of a cover portion of a lens carrier and support portion of the apparatus of the invention, showing the cover in a closed position;
Fig. 7 is a sectional view similar to Fig. 6 and showing an intermediate position of the cover portion thereof in full line and a fully open position thereof in phantom line;
Fig. 8 is an exploded perspective view illustrating the assembly of the catalyst member and carrier member with the lens support assembly utilizing a specialized tool in accordance with an alternate form of the invention;
Fig. 9 shows a further step in the assembly indicated in Fig. 8;
Fig. 10 is an enlarged partial perspective view of a portion of the lens support structure in accordance with a preferred form of the invention;
Fig. 11 is a sectional view taken generally along the line 11-11 of Fig. 10;
Fig. 12 is a partially exploded elevational view showing an alternate form of catalyst member and catalyst carrier portion of the lens carrier and support assembly, in accordance with an alternate form of the invention;
Fig. 13 is an exploded perspective view indicating assembly of the catalyst, lens carrier and container portion in accordance with the embodiment of Fig. 12; and
Fig. 14 is a bottom plan view showing the catalyst member configured in accordance with Figs. 12 and 13 assembled with the lens carrier assembly.

### Detailed Description of the Illustrated Embodiment

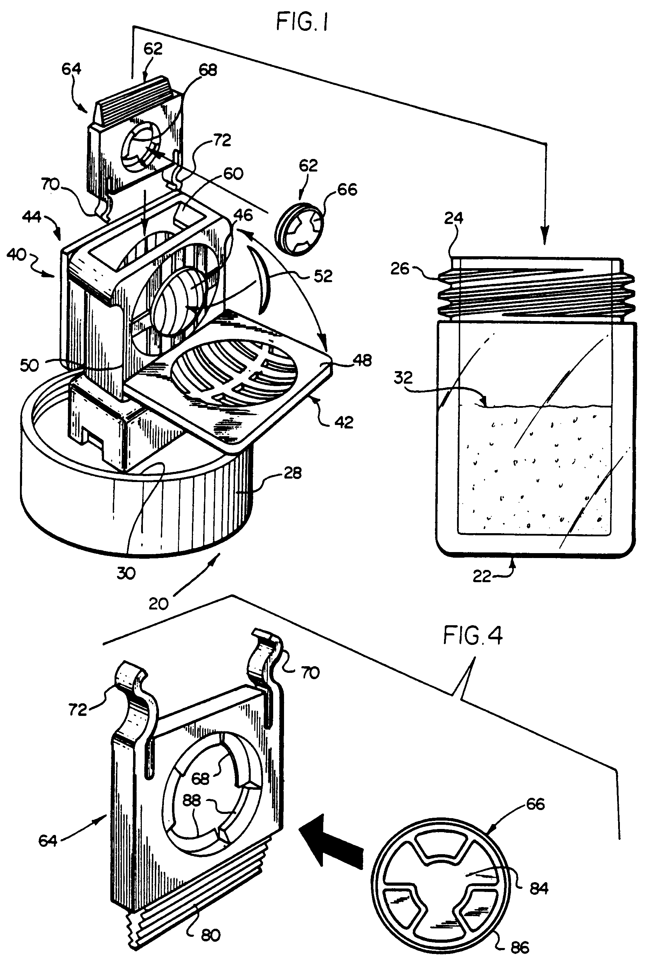

Referring initially to Figs. 1-3, the apparatus in accordance with the invention is designated generally by the reference numeral 20. Generally speaking, the apparatus 20 includes a container or reaction vessel 22, which is preferably generally cylindrical in shape and terminates in an open top 24 which preferably has a thread 26 formed thereupon for threadably engaging a complementary thread 28 formed within a cap member 30. This reaction vessel or container 22 is particularly adapted to contain a quantity of sterilizing solution 32, which preferably comprises hydrogen peroxide. In accordance with prevailing practice, the hydrogen peroxide is of a relatively low concentration, and preferably no more than a 3% solution of hydrogen peroxide in water. Different concentrations of hydrogen peroxide may be used in particular applications without departing from the invention.

In accordance with the customary practice, the cap member 30 also mounts a lens supporting structure or assembly designated generally by the reference numeral 40. The lens supporting structure 40 comprises a pair of basket-like lens-receiving or lens support structures 42, 44 which are substantially identical and oppositely facing. Each lens supporting structure comprises a base 43, 45 which includes a lens-supporting dome-like or semi-spherical member or portion 46, 47 and an overlying and complementary formed basket-like lid member 48, 49 which is preferably hingedly attached to a base or frame portion 50 from which the aforementioned lens support members 46, 47 projects.

Preferably, both the cover member 48, 49 and the lens support member or portions 46, 47 are formed of a lattice-like structure to promote flow of the fluid 32 therethrough and around contact lenses 52, 53 disposed therebetween when the cap 30 and depending lens support structure 40 are coupled with the container or reaction vessel 22. In this regard, it will be seen that the cap and lens support structure 30, 40 are illustrated in an inverted position in Fig. 1, such that in use the structure of Fig. 1 is inverted such that the threads of the cap 30 engage the threads 26 of the container 22 and such that the contact lenses, as indicated for example by reference numeral 52, are supported in a position depending from the cap and immersed within the fluid 32 in the container 22. Most of the foregoing structure is conventional in form, and need not be described in further detail herein.

Departing from convention, and in accordance with an important feature of the invention, the lens support or lens carrying structure 40 is further provided with an open-ended central interior receptacle or chamber 60. Within this receptacle 60 there is received a catalytic agent indicated generally by reference numeral 62 in a position substantially intermediate the contact lenses, e.g., 52, 53 supported within the basket-like structures defined by projection 46 and basketlike cover member 48. The mounting of the catalyst member 62 in this fashion substantially precludes any physical contact of the user with the catalytic agent during use; however, as will be seen later, the catalytic agent or catalyst member 62 is easily removable and replaceable when desired, as for example, when replacing a catalytic agent which has become exhausted from use.

Advantageously, the placing of the catalyst member or catalytic agent 62 within the lens support structure in this manner achieves two important ends. Firstly, this arrangement substantially prevents contact of the catalyst material by the user, thereby preventing any undesirable effect on the catalyst, for example the formation of an inert compound on the catalyst agent as a result of contact with sulfur compounds and proteins in the skin. As a second important matter, the placement of the catalyst or catalytic agent in the lens carrying structure prevents the catalyst from coming into contact with the solution 32 until the user has completed the placement of the lenses within the lens support structure, inverted the cap and lens support structure, and placed the same within the container 22 to begin the sterilization process.

In some prior art apparatus, a catalyst member is placed directly into the container 22, thus beginning the breakdown of the sterilizing solution prior to the actual introduction of the contact lenses for sterilization therein. In the event the user elects to fill the container 22, with the catalyst in place therein, with the sterilizing solution 32 prior to completing the placement of the contact lenses on the lens support structure, or in the event of any other delay between the beginning of contact of the catalyst with the solution and the actual placement of the lens carrying structure within the container 22, the breakdown of the sterilizing solution may be undesirably accelerated. Accordingly, the structure of the present invention helps to insure a maximum time of contact of the lenses with a maximum strength sterilizing solution initially, and avoids a premature breakdown of the sterilizing solution due to premature contact with the catalyst or catalytic agent.

In a preferred embodiment illustrated in Figs. 1-5, the means for mounting the catalyst or catalytic agent within the lens support structure further comprises a carrier member 64 which has an enlarged, frame-like body. A disc-like catalyst carrying member or portion or "disc" 66 receives a quantity of catalyst material thereon; for example, by a sputter coating or other deposition process. The disc 66 is complementary in form for being releasably supported and mounted upon the frame-like member 64 and, more particularly, within a complementary opening 68 formed in the frame-like member 64.

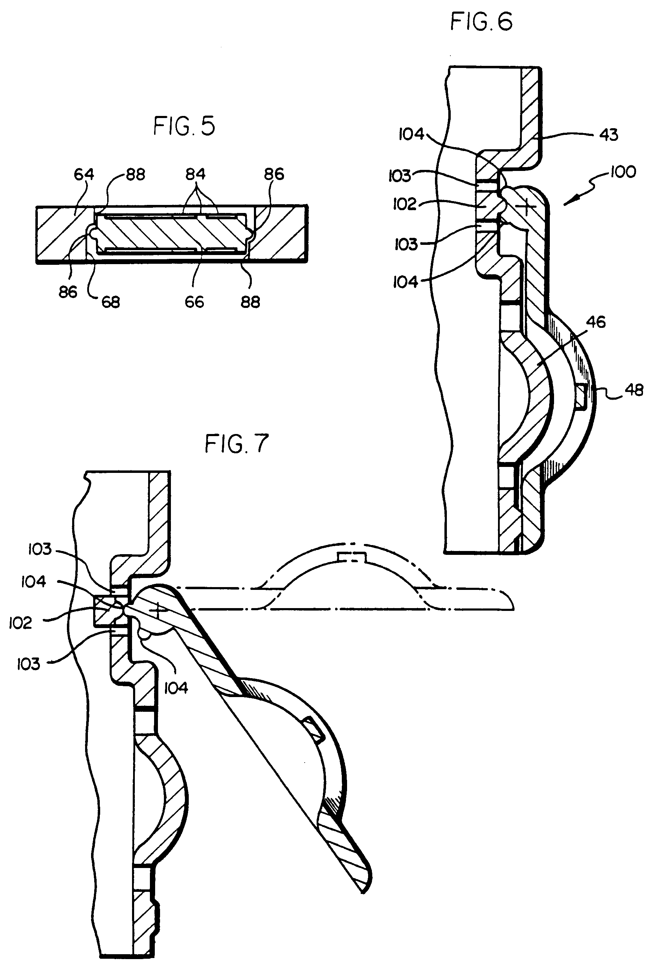

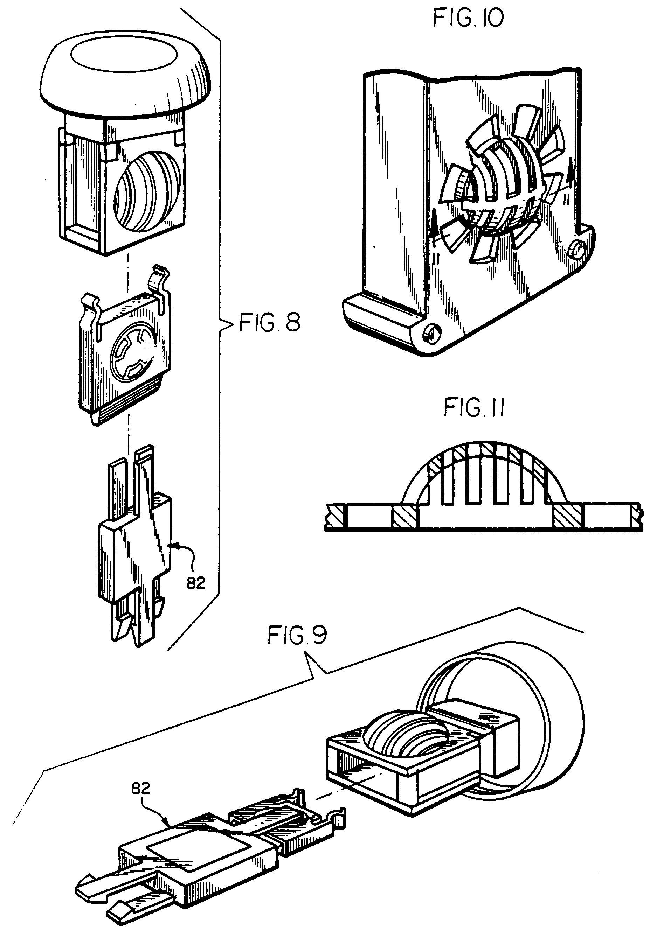

Referring now briefly to Figs. 4 and 5, it will be seen that the frame-like carrier member 64 includes a pair of resilient clip-like engaging or locking members 70, 72. Cooperatively, an interior portion of the receptacle 60 includes complementary mating projections 74, 76 for releasably engaging the resilient clip-like engaging members. Preferably the frame-like carrier member 64 also includes a projecting and preferably serrated gripping portion 80 which may be gripped by the user for inserting and/or removing the same relative to the receptacle 60. Alternatively, as shown in Figs. 8 and 9, a separate insertion/removal tool 82 may be provided for this purpose.

As best viewed in Figs 4 and 5, and in accordance with a preferred form of the invention, the catalyst disc 66 comprises a generally flat, disc-like member with a pattern of recesses and ridges 84 formed on either face or surface thereof. These recesses and ridges generally aid in the process of manufacture and plating or coating of the disc member 66 with catalyst. In order to secure the disc 66 within the complementary opening 68 in carrier 64, a projecting annular rim 86 is formed around the periphery of the disc 66. This rim 86 is engaged by a series of tabs 88 which project from alternate sides of the opening 68 in the embodiment illustrated in Figs. 4 and 5, to provide for a snapping engagement therebetween when the disc is inserted from either side.

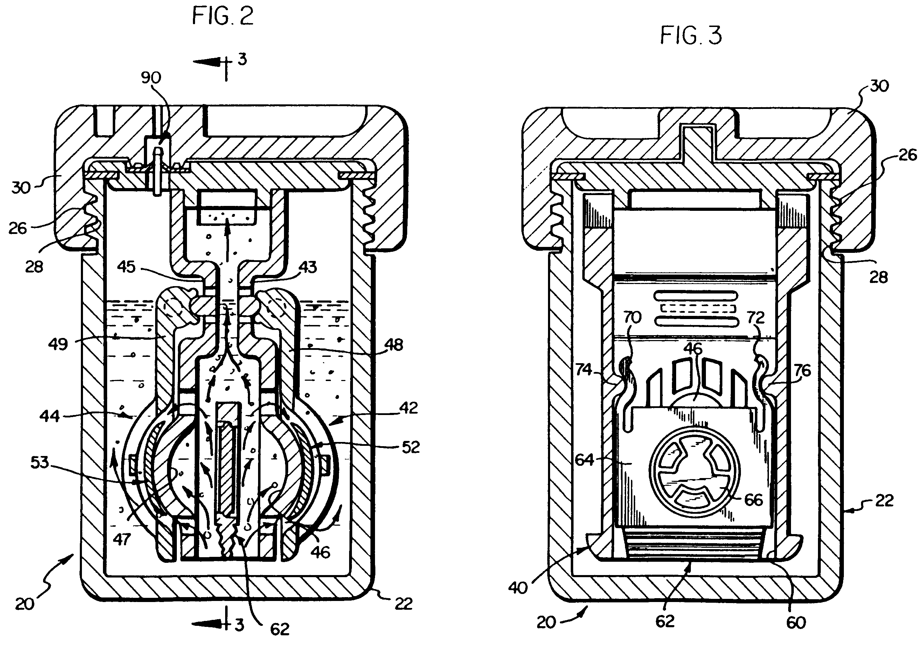

As best viewed in Fig. 2, the cap 30 is also provided with a venting structure designated generally by the reference numeral 90, which is the same as the venting structure which is more fully disclosed and described in copending application of R. W. Kanner et al, Serial No. 268,053 filed November 7, 1988 which is commonly owned with this application, and to which reference is invited concerning further details of the venting structure 90. Venting structure 90 permits the buildup of a controlled amount of pressure due to the release of oxygen in the decomposition of the hydrogen peroxide. While this oxygen is not necessarily vented through the venting structure 90, excess pressure in the vessel is nonetheless prevented by the release of some amount of the residual air and/or added oxygen in the space between the level of filling of the container with hydrogen peroxide and the cap.

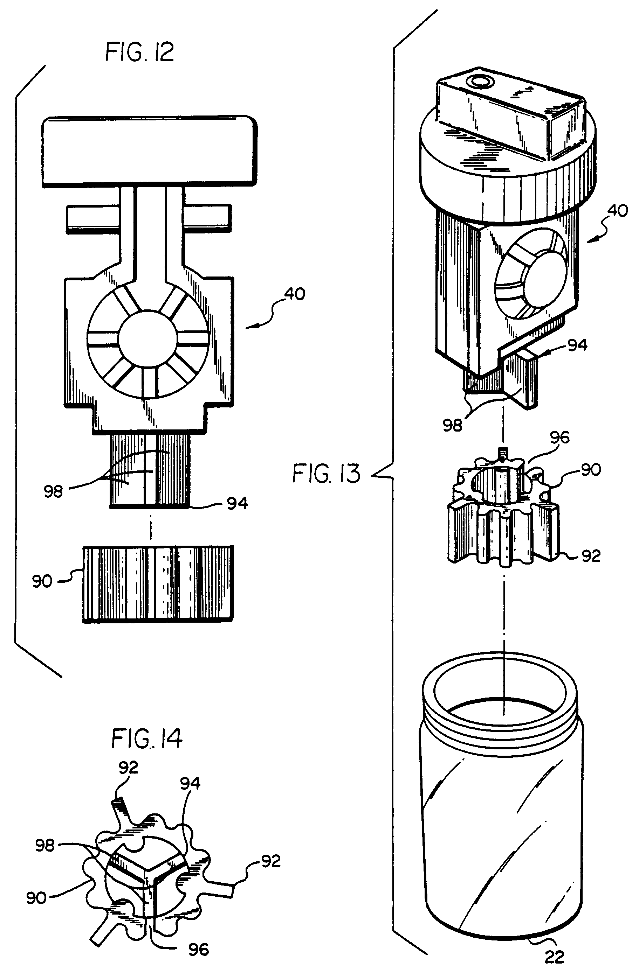

Referring next to Figs. 12-14, an alternative embodiment of the mounting means on the lens carrier structure for receiving a catalyst member or catalytic agent is shown. In the embodiment of Figs. 12-14, the catalytic agent is similar if not identical in form to a presently used catalytic agent and comprises a generally hollow, and circular, cogwheel-shaped body 90. The body 90 is shaped to cooperatively fit within the bottom portion of the cylindrical container 22. To this end the cogwheel-shaped body 90, as best viewed in Figs. 13 and 14 has three radially outwardly projecting spines 92 which serve to position the body 90 relative to the interior walls of cylindrical container 22.

Departing from conventional practice, the body 90 is not placed within the container 22 by the user. Rather, the lens supporting structure 40 is provided with an additional projecting catalyst-engaging portion 94 which engages with and carries the catalyst member 90. In this regard the catalyst member 90 has a small gap or opening 96 therein, which imparts a somewhat resilient nature thereto, such that the catalyst member 90 may be resiliently expanded somewhat to interfit over the projection 94 in a friction-type fit. In the embodiment illustrated in Figs. 12-14, the projecting member 94 is a three-legged member having three substantially equal and equi-angularly spaced radial projections 98, which are dimensioned for gripping engagement with an interior surface of the catalyst member 90. Accordingly, the catalyst member 90 is engageable with the projection 94 so as to be carried upon the lens support structure 40, such that the catalyst will not be introduced into the solution within copntainer 22 prior to the introduction of the lenses carried on the lens support member. However, the catalytic agent is readily removable for replacement by a user, as the catalytic agent becomes exhausted in use.

As best viewed in Figs. 6 and 7, the lens supporting structure also includes means 100 defining a detent arrangement for holding each cover 48, 49 detented in its respective fully open and fully closed positions relative to the associated lens sjupport member 46, 47. In the illustrated embodiment, this detenting arrangement comprises a resiliently deflectable protruding member 102, which is formed in the base 43, 45 at a position oppositely facing and aligned with an upper edge portion of the cover, at which portion the cover is hingedly coupled with the base. This protruding resilient member is formed by cutting a pair of parallel elongate thin through openings 103 in the base 43, 45 and forming a protruding, bump-like projection or portion therebetween. Cooperatively, the cover member 45 includes at least one projection or bump-like portion 104, which engages this bump-like portion during the opening and closing of the cover, and thereby defines a detented position of the cover to either side of the area or distance over which the respective projections 102, 104 engage during opening and closing thereof.

While particular embodiments of the invention have been shown and described in detail, it will be obvious to those skilled in the art that changes and modifications of the present invention, in its various aspects, may be made without departing from the invention in its broader aspects, some of which changes and modifications being matters of routine engineering or design, and others being apparent only after study. As such, the scope of the invention should not be limited by the particular embodiment and specific construction described herein but should be defined by the appended claims and equivalents thereof.

## Claims

1. Apparatus for use in the sterilization of contact lenses or the like, wherein said lenses are disposed within a sterilizing solution contained within a reaction vessel, which sterilizing solution is capable of being decomposed through contact with a catalytic agent, said apparatus comprising: a reaction vessel capable of containing a sterilizing solution and comprising a container portion, cover means and means for supporting an article in contact with sterilizing solution in said container; and further including means for mounting a catalytic agent on said supporting means such that said catalhytic agent is removable for replacement by a user as the catalytic agent becomes exhausted in use; said mounting of said catalytic agent on said supporting means thereby preventing contact of the catalhytic agent with the solution in said reaction vessel until the article supporoting means with the contact lenses therein is placed in the solution in the container for beginning the sterilizing process, to thereby prevent premature breakdown of the sterilizing solution prior to the introduction of contact lenses for sterilization therein.

2. Apparatus according to claim 1 wherein said mounting means comprises receptacle means formed in said article supporting means for mounting said catalytic agent in a position substantially intermediate a pair of contact lenses supported therewith, such that phisical contact of the user with said catalytic agent during use is substantially precluded.

3. Apparatus according to claim 2 wherein said mounting means further comprises a carrier member having an enlarged, frame-like body portion configured for engaging and supporting said catalytic agent and for removable insertion relative to said receptacle means.

4. Apparatus according to claim 3 wherein said carrier member further includes resilient clip-like engaging members, and wherein said article supporting means comprises projection means in said receptacle means for releasable mating engagement with said engaging members therewithin, so as to substantially avoid manual contact with the catalytic agent during insertion and removal of the carrier relative to said receptacle means by the user.

5. Apparatus according to claim 1 wherein said mounting means comprises a projecting portion on said article supporting means, having a complementary form with said catalytic agent for releasably engaging the same.

6. Apparatus according to claim 1 wherein said article supporting means includes a lens supporting base member and a cover member hingedly coupled for surroundingly enclosing a lens with respect to said lens-receiving base, and detent means formed on said cover member and said base respectively for defining detented positions of said cover in respective fully opened and fully closed positions thereof.

7. Apparatus according to claim 2 wherein said lens support surfaces define a basket-like structure presenting a plurality of through openings for the passage of said solution for contacting and cleaning a contact lens supported thereupon.

8. Apparatus according to claim 7 wherein said article supporting means further includes a basket-like lid member overlying and of complementary form with said lens supporting surface for overlying and surroundingly covering a contact lens supported on said surface, said basket-like lid member also presenting a plurality of through openings for the passage of said solution therethrough and said article supporting means defining through openings for placing said receptacle means in communication with said container adjacent said cover means thereof for permitting the free flow of said solution and of oxygen released in the decomposition thereof around and through said article supporting means to encourage intimate contact of the solution with said contact lenses for cleaning thereof.

9. Apparatus for use with a catalytic agent in the sterilization of contact or the like, wherein said lenses are disposed within a sterilizing solution contained within a reaction vessel, which sterilizing solution is capable of being decomposed through contact with said catalytic agent, said apparatus comprising: a reaction vessel capable of containing a sterilizing solution and comprising a container portion, cover means and article supporting means including article support surfaces for supporting said contact lenses in contact with sterilizing solution in said container; and further including means for mounting said catalytic agent on said article supporting means such that said catalytic agent is removable for replacement by a user as the catalytic agent becomes exhausted in use; said mounting means comprising receptacle means formed in said article supporting means for mounting said catalytic agent in a position substantially intermediate said article support surfaces, such that physical contact of the user with said catalytic agent during use is substantially precluded; said mounting of said catalytic agent in said receptacle means thereby preventing contact of the catalytic agent with the solution in said reaction vessel until the article supporting means with the contact lenses therein is placed in the solution in the container for beginning the sterilizing process, to thereby prevent premature breakdown of the sterilizing solution prior to the introduction of contact lenses for sterilization therein.

10. Apparatus according to claim 9 wherein said mounting means further comprises a carrier member having an enlarged, frame-like body portion configured for engaging and supporting said catalytic agent, and for removable insertion relative to said receptacle means.

11. Apparatus according to claim 10 wherein said carrier member further includes resilient clip-like engaging members, and wherein said article supporting means comprises mating projection means in said receptacle means for releasable engagement with said engaging members therewithin, so as to substantially avoid manual contact with the catalytic agent during insertion and removal of the carrier relative to said receptacle means by the user.

12. Apparatus according to claim 9 wherein said article supporting means includes a lens supporting base member and a cover member hingedly coupled for surroundingly enclosing a lens with respect to said lens-receiving base, and detent means formed on said cover member and said base respectively for defining detented positions of said cover in respective fully opened and fully closed positions thereof.

13. Apparatus according to claim 9 wherein said lens support surfaces define a basket-like structure presenting a plurality of through openings for the passage of said solution for contacting and cleaning a contact lens supported thereupon.

14. Apparatus according to claim 13 wherein said article supporting means further includes a basket-like lid member overlying and of complementary form with said lens supporting surface for overlying and surroundingly covering a contact lens supported on said surface, said basket-like lid member also presenting a plurality of through openings for the passage of said solution therethrough and said article supporting means defining through openings for placing said receptacle means in communication with said container adjacent said cover means thereof for permitting the free flow of said solution and of oxygen released in the decomposition thereof around and through said article supporting means to encourage intimate contact of the solution with said contact lenses for cleaning thereof.

15. Apparatus for use with a catalytic agent in the sterilization of contact lenses or the like, wherein said lenses are disposed within a sterilizing solution contained within a reaction vessel, which sterilizing solution is capable of being decomposed through contact with said catalytic agent, said apparatus comprising: a reaction vessel capable of containing a sterilizing solution and comprising a container portion, cover means and article supporting means for supporting a contact lens in contact with sterilizing solution in said container; and further including a projecting portion on said article supporting means, having a complementary form with said catalytic agent for releasably engaging the same for mounting said catalytic agent on said supporting means such that said catalytic agent is removable for replacement by a user as the catalytic agent becomes exhausted in use; said mounting of said catalytic agent on said article supporting means thereby preventing contact of the catalytic agent with the solution in said container until the article supporting means with the contact lenses therein is placed in the solution in the reaction vessel for beginning the sterilizing process, to thereby prevent premature breakdown of the sterilizing solution prior to the introduction of contact lenses for sterilization therein.

16. Apparatus for use in the sterilization of contact lenses or the like, wherein said lenses are disposed within a sterilizing solution contained within a reaction vessel, which sterilizing solution is capable of being decomposed through contact with a catalytic agent, said apparatus comprising: a reaction vessel capable of containing a sterilizing solution and comprising a container portion, cover means and article supporting means for supporting a contact lens in contact with sterilizing solution in said container; a catalytic agent comprising a catalyst-receiving body of predetermined shape coated with a quantity of catalyst material; and further including means for mounting said catalytic agent on said article supporting means such that said catalytic agent is removable for replacement by a user as the catalytic agent becomes exhausted in use; said mounting of said catalytic agent on said article supporting means thereby preventing contact of the catalytic agent with the solution in said reaction vessel until the article supporting means with the catalytic agent mounted thereto is placed in the solution in the container for beginning the sterilizing process, to thereby prevent premature breakdown of the sterilizing solution prior to the introduction of contact lenses for sterilization therein.

17. Apparatus according to claim 16 wherein said article supporting means includes lens support surfaces for supporting a pair of contact lenses and wherein said mounting means comprises receptacle means formed in said article supporting means for mounting said catalytic agent in a position substantially intermediate said lens support surfaces, such that physical contact of the user and of the lenses with said catalytic agent during use is substantially precluded.

18. Apparatus according to claim 17 wherein said mounting means further comprises a carrier member having an enlarged, frame-like body portion and wherein said catalyst-receiving body is shaped to be engaged and supported by said frame-like body portion.

19. Apparatus according to claim 18 wherein said carrier member further includes resilient clip-like engaging members, and wherein said article supporting means comprises projection means in said receptacle means for releasable mating engagement with said engaging members therewithin.

20. Apparatus according to claim 16 wherein said mounting means comprises a projecting portion on said carrier member, having a complementary form with said catalyst-receiving body for releasably engaging the same.

21. Apparatus according to claim 6 wherein said detent means comprises a resiliently deflectable protruding member formed in said base member at a position oppositely facing and aligned with an edge portion of the cover member, and at least one projection formed in said cover member which engages said resilient protruding member during opening and closing of the cover to define a detented position of the cover to either side of the area over which the resilient protruding member and projection engage during opening and closing of the cover.

22. Apparatus for use in the sterilization of contact lenses or the like, wherein said lenses are disposed within a sterilizing solution contained within a reaction vessel, which sterilizing solution is capable of being decomposed through contact with a catalytic agent, said apparatus comprising: article supporting means for supporting one or more contact lenses; mounting means for mounting a catalytic agent on said article supporting means such that said catalytic agent is removable for replacement by a user as the catalytic agent becomes exhausted in use; said mounting means comprising a body portion and resilient grasping means coupled with said body portion and cooperating mounting means formed on said article supporting means for engaging said resilient grasping means for removably mounting said mounting means relative to said article supporting means; said body portion being coated with a quantity of catalyst material; said mounting of said catalytic agent on said article supporting means thereby preventing contact of the catalytic agent with the solution in said container until the article supporting means with the catalytic agent mounted thereon is placed in the solution in the reaction vessel for beginning the sterilizing process, to thereby prevent premature breakdown of the sterilizing solution prior to the introduction of contact lenses for sterilization therein.

23. A catalyst apparatus for use in the sterilization of contact lenses or the like, wherein said lenses are disposed in article supporting means within a sterilizing solution contained within a reaction vessel, which sterilizing solution is capable of being decomposed through contact with a catalytic agent, said catalyst apparatus comprising: mounting means for mounting a catalytic agent on said article supporting means such that said catalytic agent is removable for replacement by a user as the catalytic agent becomes exhausted in use; said mounting means comprising a body portion and snap-in clip means coupled with said body portion and configured for engaging cooperating mounting means formed on said article supporting means for removable mounting relative to said article supporting means; said body portion being coated with a quantity of catalyst material, and an insertion/removal tool having engaging means formed for engaging and releasing said body portion for mounting relative to said article supporting means and for engaging said body portion for removal from said article supporting means; said mounting of said catalytic agent on said article supporting means thereby preventing contact of the catalytic agent with the solution in said container until the article supporting means with the catalyst apparatus mounted thereon is placed in the solution in the reaction vessel for beginning the sterilizing process, to thereby prevent premature breakdown of the sterilizing solution prior to the introduction of contact lenses for sterilization therein.

24. A catalyst apparatus for use in the sterilization of contact lenses or the like, wherein said lenses are disposed in article supporting means within a sterilizing solution contained within a reaction vessel, which sterilizing solution is capable of being decomposed through contact with a catalytic agent, said catalyst apparatus comprising: a catalytic agent comprising a catalyst-receiving body of predetermined shape coated with a quantity of catalyst material; and further including means for mounting said catalytic agent on said article supporting means such that said catalytic agent is removable for replacement by a user as the catalytic agent becomes exhausted in use; said mounting means comprising a carrier member having an enlarged, frame-like body portion and wherein said catalyst-receiving body is shaped to be engaged and supported by said frame-like body portion; said mounting of said catalytic agent on said article supporting means thereby preventing contact of the catalytic agent with the solution in said container until the article supporting means with the catalytic agent mounted thereto is placed in the solution in the container for beginning the sterilizing process, to thereby prevent premature breakdown of the sterilizing solution prior to the introduction of contact lenses for sterilization therein.

25. Apparatus according to claim 24 wherein said carrier member further includes resilient clip-like engaging members, located and configured for releasable mating engagement with said article supporting means.

26. Lens carrier apparatus for use with a catalytic agent in the sterilization of contact lenses or the like, wherein said lenses are disposed within a sterilizing solution contained within a reaction vessel, which sterilizing solution is capable of being decomposed through contact with said catalytic agent, said lens carrier apparatus comprising: article supporting structure for supporting a contact lens in contact with sterilizing solution in said reaction vessel; said article supporting structure including a lens supporting base member and a cover member hingedly coupled with said base member for surroundingly enclosing a lens with respect to said lens-receiving base member, and detent means formed on said cover member and said base member respectively for defining detented positions of said cover member in respective fully opened and fully closed positions thereof; wherein said detent means comprises a resiliently deflectable protruding member formed in said base member at a position oppositely facing and aligned with at least a portion of the cover member, and at least one projection formed in said cover member which engages said resilient protruding member during opening and closing of the cover member to define a detented position of the cover member to either side of the area over which the resilient protruding member and projection engage during opening and closing of the cover member.

## Patentansprüche

1. Vorrichtung zur Verwendung bei der Sterilisation von Kontaktlinsen oder ähnlichem, bei der die genannten Linsen in eine in einem Reaktionsgefäß befindliche Sterilisationslösung eingelegt werden und diese Sterilisationslösung durch Kontakt mit einem katalytischen Mittel abgebaut werden kann, wobei die genannte Vorrichtung folgendes umfaßt: ein Reaktionsgefaß, das eine Sterilisationslösung aufnehmen kann und einen Behälterteil, Abdeckmittel und Mittel umfaßt, um einen mit der Sterilisationslösung in dem genannten Behälter in Kontakt befindlichen Gegenstand zu tragen; weiter umfaßt die Vorrichtung Mittel, um in das genannte Tragmittel zur Aufnahme eines Gegenstands ein katalytisches Mittel einzusetzen, so daß dieses katalytische Mittel zum Auswechseln von einem Benutzer entnommen werden kann, wenn es verbraucht ist; wobei durch das genannte Einsetzen des katalytischen Mittels in das genannte Tragmittel der Kontakt des katalytischen Mittels mit der Lösung in dem genannten Reaktionsgefäß verhindert wird, bis das Tragmittel mit den darin befindlichen Kontaktlinsen zu Beginn des Sterilisationsprozesses in die Lösung in dem Behälter eingesetzt wird, so daß verhindert wird, daß die Sterilisationslösung bereits vor dem Einlegen der Kontaktlinsen abgebaut wird.

2. Vorrichtung nach Anspruch 1, bei der das genannte Einsetzmittel Aufnahmemittel umfaßt, die in das genannte Tragmittel geformt sind, um das genannte katalytische Mittel in einer Position anzubringen, die im wesentlichen zwischen einem Paar von in das Tragmittel eingelegten Kontaktlinsen liegt, so daß ein physischer Kontakt des Benutzers mit dem genannten katalytischen Mittel beim Gebrauch im wesentlichen ausgeschlossen ist.

3. Vorrichtung nach Anspruch 2, bei der das genannte Einsetzmittel weiter ein Trägerelement mit einem vergrößerten rahmenähnlichen Gehäuseteil umfaßt, zum Halten und Abstützen des genannten katalytischen Mittels und zum Einsetzen und Herausnehmen in das bzw. aus dem genannten Aufnahmemittel.

4. Vorrichtung nach Anspruch 3, bei der das genannte Tragerelement weiter elastische bügelartige Eingriffsmittel umfaßt, und bei der das genannte Tragmittel in dem genannten Aufnahmemittel vorspringende Mittel hat, die zu den genannten Eingriffsmitteln passen, und mit diesen wieder lösbar verbunden werden können, so daß ein manueller Kontakt des Benutzers mit dem katalytischen Mittel beim Einlegen und Herausnehmen des Trägers in das genannte Aufnahmemittel im wesentlichen vermieden wird.

5. Vorrichtung nach Anspruch 1, bei der das genannte Einsetzmittel einen vorspringenden Teil an dem genannten Tragmittel zur Aufnahme des Gegenstands umfaßt, der zu dem genannten katalytischen Mittel eine komplementäre Form hat, um dasselbe wieder lösbar festzuhalten.

6. Vorrichtung nach Anspruch 1, bei das genannte Tragmittel zur Aufnahme eines Gegenstands ein Grundelement zur Aufnahme der Linse und ein schwenkbar gekoppeltes Abdeckelement umfaßt, das eine Linse in Verbindung mit dem genannten Grundelement rundum abschließt, und an dem genannten Abdeckelement bzw. dem genannten Grundelement Feststellmittel geformt sind, um in der jeweils voll geöffneten und voll geschlossenen Stellung des genannten Abdeckmittels Feststellpositionen dieses Abdeckmittels festzulegen.

7. Vorrichtung nach Anspruch 2, bei der die genannten Linsen-Aufnahmeflächen eine körbchenartige Struktur aufweisen, mit einer Vielzahl von durchgehenden Öffnungen, durch die die genannte Lösung hindurchtreten kann, um mit der auf dieser Fläche liegenden Kontaktlinse bei der Reinigung in Kontakt zu kommen.

8. Vorrichtung nach Anspruch 7, bei der das genannte Tragmittel weiter ein körbchenartiges Deckelelement aufweist, das über der genannten Linsen-Aufnahmefläche liegt und zu dieser eine komplementäre Form aufweist, um über einer auf der genannten Fläche liegenden Kontaktlinse diese rundum abzuschließen, wobei das genannte körbchenartige Deckelelement ebenfalls eine Vielzahl von durchgehenden Öffnungen aufweist, durch die die genannte Lösung hindurchtreten kann, und das genannte Tragmittel durchgehende Öffnungen aufweist, um das genannte Aufnahmemittel in Verbindung mit dem genannten Behälter neben dem genannten Abdeckmittel dieses Behälters zu plazieren, um den freien Fluß der genannten Lösung und des beim Abbau der Lösung freiwerdenden Sauerstoffs um und durch das genannte Tragmittel zu ermöglichen und beim Reinigen für einen engen Kontakt der Lösung mit den genannten Kontaktlinsen zu sorgen.

9. Vorrichtung zur Verwendung mit einem katalytischen Mittel bei der Sterilisation von Kontaktlinsen oder ähnlichem, bei der die genannten Linsen in eine in einem Reaktionsgefäß befindliche Sterilisationslösung eingelegt werden und diese Sterilisationslösung durch Kontakt mit dem genannten katalytischen Mittel abgebaut werden kann, wobei die genannte Vorrichtung folgendes umfaßt: ein Reaktionsgefäß, das eine Sterilisationslösung aufnehmen kann und einen Behälterteil, Abdeckmittel und Tragmittel mit Flächen zur Aufnahme eines Gegenstands umfaßt, um die mit der Sterilisationslösung in Kontakt befindlichen Kontaktlinsen in dem genannten Behälter aufzunehmen; und das weiter Mittel umfaßt, um das genannte katalytische Mittel in das genannte Tragmittel einzusetzen, so daß das genannte katalytische Mittel, nachdem es verbraucht ist, von einem Benutzer entnommen und ausgewechselt werden kann; wobei das genannte Einsetzmittel Aufnahmemittel umfaßt, die in das genannte Tragmittel geformt sind, um das genannte katalytische Mittel in einer Position anzubringen, die sich im wesentlichen zwischen den Aufnahmeflächen für den Gegenstand befindet, so daß der physische Kontakt des Benutzers mit dem genannten katalytischen Mittel beim Gebrauch im wesentlichen ausgeschlossen ist; wobei durch das genannte Einsetzen des genannten katalytischen Mittels in das genannte Aufnahmemittel der Kontakt des katalytischen Mittels mit der Lösung in dem genannten Reaktionsgefäß verhindert wird, bis das Tragmittel mit den darin befindlichen Kontaktlinsen in die in dem Behälter befindliche Lösung eingelegt wird, um den Sterilisationsprozeß zu beginnen, so daß der vorzeitige Abbau der Sterilisationslösung vor dem Einlegen der Kontaktlinsen verhindert wird.

10. Vorrichtung nach Anspruch 9, bei der das genannte Einsetzmittel weiter ein Trägerelement mit einem vergrößerten rahmenähnlichen Gehäuseteil umfaßt, der das katalytische Mittel festhalten und abstützen kann, und mit dem das katalytische Mittel in das Aufnahmemittel eingesetzt und wieder entnommen werden kann.

11. Vorrichtung nach Anspruch 10, bei der das genannte Trägerelement weiter elastische bügelähnliche Eingriffselemente umfaßt, und bei der das genannte Tragmittel dazu passende vorspringende Mittel in dem genannten Aufnahmemittel aufweist, die mit den genannten Eingriffsmitteln wieder lösbar verbunden werden können, so daß im wesentlichen der manuelle Kontakt des Benutzers mit dem katalytischen Mittel beim Einlegen und Herausnehmen des Trägers in das bzw. aus dem Aufnahmemittel verhindert wird.

12. Vorrichtung nach Anspruch 9, bei der das genannte Tragmittel ein Grundelement zur Aufnahme der Linse und ein damit schwenkbar verbundenes Abdeckelement umfaßt, um eine Linse in Verbindung mit dem genannten Aufnahme-Grundelement rundum abzuschließen, und auf dem genannten Abdeckmittel bzw. dem genannten Grundelement geformte Feststellmittel zur Festlegung von Feststellpositionen der genannten Abdeckung in ihrer jeweiligen ganz geöffneten und ganz geschlossenen Stellung.

13. Vorrichtung nach Anspruch 9, bei der die genannten Linsenaufnahmeflächen eine körbchenartige Struktur aufweisen, die über eine Vielzahl von durchgehenden Öffnungen verfügt, durch die die genannte Lösung hindurchtreten kann, um mit einer darauf liegenden Kontaktlinse beim Reinigungsprozeß in Kontakt zu kommen.

14. Vorrichtung nach Anspruch 13, bei der das genannte Tragmittel zur Aufnahme eines Gegenstands weiter ein körbchenartiges Deckelelement umfaßt, das über der genannten Linsenaufnahmefläche liegt und zu dieser eine komplementäre Form hat, so daß es über einer auf der genannten Fläche liegenden Kontaktlinse liegt und diese rundum abdeckt, wobei das genannte körbchenartige Deckelelement ebenfalls eine Vielzahl von durchgehenden Öffnungen für den Durchtritt der genannten Lösung aufweist, und das genannte Tragmittel durchgehende Öffnungen aufweist, um das genannte Aufnahmemittel in Verbindung mit dem genannten Behälter neben dem genannten Abdeckmittel des Behälters anzuordnen, um den freien Fluß der genannten Lösung und des beim Abbau der Lösung freiwerdenden Sauerstoffs um und durch das genannte Tragmittel zu ermöglichen, so daß beim Reinigen ein enger Kontakt zwischen der Lösung und den genannten Kontaktlinsen entsteht.

15. Vorrichtung zur Verwendung mit einem katalytischen Mittel zur Sterilisation von Kontaktlinsen oder ähnlichem, bei der die genannten Linsen in eine in einem Reaktionsgefäß befindliche Sterilisationslösung eingelegt werden und die genannte Sterilisationslösung durch Kontakt mit dem genannten katalytischen Mittel abgebaut werden kann, wobei die genannte Vorrichtung folgendes umfaßt: ein Reaktionsgefäß, das eine Sterilisationslösung aufnehmen kann und einen Behälterteil, ein Abdeckmittel und ein Tragmittel umfaßt, um eine Kontaktlinse aufzunehmen, die sich mit der Sterilisationslösung in dem genannten Behälter in Kontakt befindet; und die weiter einen vorspringenden Teil an dem genannten Tragmittel umfaßt, der zu dem genannten katalytischen Mittel eine komplementäre Form hat, um dasselbe wieder lösbar festzuhalten, um das genannte katalytische Mittel in das genannte Tragmittel einzusetzen, so daß das genannte katalytische Mittel, wenn es verbraucht ist, von einem Benutzer entnommen und ausgewechselt werden kann; wobei das Einsetzen des genannten katalytischen Mittels in das genannte Tragmittel dadurch den Kontakt des katalytischen Mittels mit der Lösung in dem genannten Behälter verhindert, bis das Tragmittel mit den darin befindlichen Kontaktlinsen in die Lösung in dem Reaktionsgefäß eingesetzt wird, um mit dem Sterilisationsprozeß zu beginnen, so daß ein vorzeitiger Abbau der Sterilisationslösung vor dem Einlegen der Kontaktlinsen verhindert wird.

16. Vorrichtung zur Verwendung bei der Sterilisation von Kontaktlinsen oder ähnlichem, bei der die genannten Linsen in eine in einem Reaktionsgefäß befindliche Sterilisationslösung eingelegt werden und diese Sterilisationslösung durch Kontakt mit einem katalytischen Mittel abgebaut werden kann, wobei die genannte Vorrichtung folgendes umfaßt: ein Reaktionsgefäß, das eine Sterilisationslösung aufnehmen kann und einen Behälterteil, Abdeckmittel und Tragmittel umfaßt, um eine Kontaktlinse aufzunehmen, die sich mit der Sterilisationslösung in dem genannten Behälter in Kontakt befindet; ein katalytisches Mittel, umfassend einen Gehäuseteil von vorbestimmter Form zur Aufnahme eines Katalysators, der mit einer bestimmten Menge des Katalysatormaterials beschichtet ist; und weiter umfassend Mittel, um das genannte katalytische Mittel in das genannte Tragmittel einzusetzen, so daß das genannte katalytische Mittel, wenn es verbraucht ist, von einem Benutzer entnommen und ausgewechselt werden kann; wobei das genannte Einsetzen des genannten katalytischen Mittels in das genannte Tragmittel dadurch den Kontakt des katalytischen Mittels mit der Lösung in dem genannten Reaktionsgefäß verhindert, bis das Tragmittel mit dem darin eingesetzten katalytischen Mittel in die Lösung in dem Behälter eingesetzt wird, um den Sterilisationsprozeß zu beginnen, so daS ein vorzeitiger Abbau der Sterilisationslösung vor dem Einlegen der zu sterilisierenden Kontaktlinsen verhindert wird.

17. Vorrichtung nach Anspruch 16, bei der das genannte Tragmittel Linsenaufnahmeflächen aufweist, um ein Kontaktlinsenpaar aufzunehmen, und bei der das genannte Einsetzmittel Aufnahmemittel umfaßt, die in das genannte Tragmittel geformt sind, um das genannte katalytische Mittel in einer Position anzubringen, die im wesentlichen zwischen den genannten Linsenaufnahmeflächen liegt, so daS ein physischer Kontakt des Benutzers und der Linsen mit dem genannten katalytischen Mittel beim Gebrauch im wesentlichen ausgeschlossen ist.

18. Vorrichtung nach Anspruch 17, bei der das genannte Einsetzmittel weiter ein Trägerelement mit einem vergrößerten rahmenähnlichen Gehäuseteil umfaßt, und bei der der genannte Teil, der den Katalysator aufnimmt, so geformt ist, daS er von dem genannten rahmenähnlichen Gehäuseteil festgehalten und abgestützt werden kann.

19. Vorrichtung nach Anspruch 18, bei der das genannte Trägerelement weiter elastische bügelähnliche Eingriffsmittel umfaßt, und bei der das genannte Tragmittel vorspringende Mittel in dem genannten Aufnahmemittel aufweist, um mit den zu diesen passenden Eingriffsmitteln eine wieder lösbare Verbindung herstellen zu können.

20. Vorrichtung nach Anspruch 16, bei der das genannte Einsetzmittel einen vorspringenden Teil an dem genannten Trägerelement umfaßt, der zu dem genannten Teil zur Aufnahme des Katalysators eine komplementäre Form hat, um diesen wieder lösbar festzuhalten.

21. Vorrichtung nach Anspruch 6, bei der das genannte Feststellmittel ein in dem genannten Grundelement geformtes elastisch auslenkbares vorstehendes Element umfaßt, in einer Position, die einem Kantenteil des Abdeckelements gegenüberliegt und mit diesem ausgerichtet ist, und mindestens einen in dem genannten Abdeckmittel geformten Vorsprung, der in das genannte elastische vorstehende Element beim Öffnen und Schließen der Abdeckung eingreift, um eine Feststellposition der Abdeckung zu beiden Seiten des Bereichs zu definieren, über dem das elastische vorspringende Element und der Vorsprung beim Öffnen und Schließen des Deckels ineinandergreifen.

22. Vorrichtung zur Verwendung bei der Sterilisation von Kontaktlinsen oder ähnlichem, bei der die genannten Linsen in eine Sterilisationslösung in einem Reaktionsgefäß eingelegt werden und diese Sterilisationslösung durch Kontakt mit einem katalytischen Mittel abgebaut werden kann, wobei die genannte Vorrichtung folgendes umfaßt: Tragmittel zur Aufnahme einer oder mehrerer Kontaktlinsen; Einsetzmittel zum Einsetzen eines katalytischen Mittels in das genannte Tragmittel, so daß das genannte katalytische Mittel von einem Benutzer entnommen und ausgewechselt werden kann, wenn es verbraucht ist; wobei das genannte Einsetzmittel einen Gehäuseteil und ein elastisches, mit dem genannten Gehäuseteil gekoppeltes Festhaltemittel umfaßt, und damit zusammenwirkende Einsetzmittel, die in das genannte Tragmittel geformt sind, um das genannte elastische Festhaltemittel zu halten, so daß das genannte Einsetzmittel in das genannte Tragmittel eingesetzt und wieder entnommen werden kann; wobei der genannte Gehäuseteil mit einer bestimmten Menge Katalysatormaterial beschichtet ist; das Einsetzen des genannten katalytischen Mittels in das genannte Tragmittel dadurch den Kontakt des katalytischen Mittels mit der Lösung in dem genannten Behälter verhindert, bis das Tragmittel mit dem darin eingesetzten katalytischen Mittel in die Lösung in dem Reaktionsgefäß eingesetzt wird, um den Sterilisationsprozeß zu beginnen, so daß ein vorzeitiger Abbau der Sterilisationslösung vor dem Einlegen der Kontaktlinsen in die Lösung verhindert wird.

23. Katalysatorvorrichtung zur Verwendung bei der Sterilisation von Kontaktlinsen oder ähnlichem, bei der die genannten Linsen in einem Tragmittel in eine Sterilisationslösung in einem Reaktionsgefäß eingelegt werden und diese Sterilisationslösung durch Kontakt mit dem katalytischen Mittel abgebaut werden kann, wobei die genannte Katalysatorvorrichtung folgendes umfaßt: Einsetzmittel zum Einsetzen eines katalytischen Mittels in das genannte Tragmittel, so daß das genannte katalytische Mittel von einem Benutzer entnommen und ausgewechselt werden kann, wenn es verbraucht ist; wobei das genannte Einsetzmittel einen Gehäuseteil umfaßt und ein Schnapp-Bügelelement` das mit dem genannten Gehäuseteil gekoppelt ist, und in damit zusammenwirkende Einführmittel eingreifen kann, die in das genannte Tragmittel geformt sind, und in das genannte Tragmittel eingeführt und wieder entnommen werden können; wobei der genannte Gehäuseteil mit einer bestimmten Menge Katalysatormaterial beschichtet ist, und eine Einsetz-/Entnahmevorrichtung mit Eingriffsmitteln, die zum Festhalten und Lösen des genannten Gehäuseteils geformt sind, zum Einsetzen in das genannte Tragmittel und zum Halten des genannten Gehäuseteils bei der Entnahme aus dem genannten Tragmittel; wobei das genannte Einsetzen des genannten katalytischen Mittels in das genannte Tragmittel dadurch den Kontakt des katalytischen Mittels mit der Lösung in dem genannten Behälter verhindert, bis das Tragmittel mit der darin eingesetzten Katalysatorvorrichtung in die Lösung in dem Reaktionsgefäß eingesetzt wird, um den Sterilisationsprozeß zu beginnen, so daß ein vorzeitiger Abbau der Sterilisationslösung vor dem Einlegen der Kontaktlinsen verhindert wird.

24. Katalysatorvorrichtung zur Verwendung bei der Sterilisation von Kontaktlinsen oder ähnlichem, bei der die genannten Linsen in einem Tragmittel in eine in einem Reaktionsgefäß befindliche Sterilisationslösung eingelegt werden und diese Sterilisationslösung durch Kontakt mit einem katalytischen Mittel abgebaut werden kann, wobei die genannte Katalysatorvorrichtung folgendes umfaßt: ein katalytisches Mittel, einen Gehäuseteil von vorbestimmter Form zur Aufnahme eines Katalysators umfassend, der mit einer bestimmten Menge Katalysatormaterial beschichtet ist; und weiter Mittel umfassend, um das genannte katalytische Mittel in das genannte Tragmittel einzusetzen, so daß das genannte katalytische Mittel vom Benutzer entnommen und ausgewechselt werden kann, wenn es verbraucht ist; wobei das genannte Einsetzmittel ein Trägerelement mit einem vergrößerten rahmenähnlichen Gehäuseteil umfaßt, bei dem der genannte Teil zur Aufnahme des Katalysators so geformt ist, daß er in den genannten rahmenähnlichen Gehäuseteil eingreift und von diesem abgestützt wird; wobei das genannte Einsetzen des genannten katalytischen Mittels in das genannte Tragmittel dadurch den Kontakt des katalytischen Mittels mit der Lösung in dem genannten Behälter verhindert, bis das Tragmittel mit dem darin eingesetzten katalytischen Mittel in die Lösung in dem Behälter eingelegt wird, um den Sterilisationsprozeß zu beginnen, so daß ein vorzeitiger Abbau der Sterilisationslösung vor dem Einlegen der Kontaktlinsen verhindert wird.

25. Vorrichtung nach Anspruch 24, bei der das genannte Trägerelement weiter elastische bügelähnliche Eingriffsmittel umfaßt, die so angeordnet und beschaffen sind, daß sie mit dem genannten Tragmittel passend in Eingriff gebracht werden können.

26. Linsentragvorrichtung zur Verwendung mit einem katalytischen Mittel bei der Sterilisation von Kontaktlinsen oder ähnlichem, bei der die genannten Linsen in eine in einem Reaktionsgefäß befindliche Sterilisationslösung eingelegt werden und diese Sterilisationslösung durch Kontakt mit dem genannten katalytischen Mittel abgebaut werden kann, wobei die genannte Linsentragvorrichtung folgendes aufweist: eine Tragkonstruktion zur Aufnahme einer Kontaktlinse, die sich mit der Sterilisationslösung in dem genannten Reaktionsgefäß in Kontakt befindet; wobei die genannte Tragkonstruktion ein Grundelement zur Aufnahme der Linse und ein mit diesem schwenkbar gekoppeltes Abdeckelement umfaßt, um eine Linse in Verbindung mit dem genannten Aufnahme-Grundelement rundum abzuschließen, und an dem genannten Abdeckelement beziehungsweise dem genannten Grundelement geformte Feststellmittel, um Feststellpositionen des genannten Abdeckelements in seiner jeweils voll geöffneten und voll geschlossenen Position festzulegen; bei der das genannte Feststellmittel ein elastisch auslenkbares vorstehendes Element umfaßt, das in dem genannten Grundelement in einer Position gegenüber dem Abdeckelement geformt und mit mindestens einem Teil des Abdeckelements ausgerichtet ist, und mindestens einen Vorsprung, der in dem genannten Abdeckelement geformt ist, und der in das genannte elastische vorstehende Element beim Öffnen und Schließen des Abdeckelements eingreift, um eine Feststellposition des Abdeckelements zu beiden Seiten des Bereichs festzulegen, über dem das elastische vorstehende Element und der Vorsprung beim Öffnen und Schließen des Abdeckelements ineinandergreifen.

## Revendications

1. Appareil prévu pour être utilisé dans la stérilisation de lentilles de contact ou similaires, dans lequel lesdites lentilles sont placées dans une solution de stérilisation contenue dans une cuve de réaction, laquelle solution de stérilisation peut se décomposer au contact d'un agent catalytique, ledit appareil comprenant: une cuve de réaction pouvant contenir une solution de stérilisation et comprenant une partie de récipient, un moyen formant couvercle et un moyen pour supporter un article en contact avec la solution de stérilisation dans ledit récipient; et comprenant, en outre, des moyens pour fixer un agent catalytique sur lesdits moyens de support de telle sorte que ledit agent catalytique peut être retiré pour être remplacé par un utilisateur lorsque l'agent catalytique est épuisé lors de son utilisation; ladite fixation dudit agent catalytique sur ledit moyen de support évitant ainsi tout contact de l'agent catalyseur avec la solution dans ladite cuve de réaction jusqu'à ce que le moyen de support de l'article avec lesdites lentilles de contact soit placé dans la solution dans le récipient pour commencer le processus de stérilisation, pour éviter ainsi toute décomposition prématurée de la solution de stérilisation avant l'introduction des lentilles à stériliser dans celle-ci.

2. Appareil selon la revendication 1, dans lequel ledit moyen de fixation comprend un moyen formant réceptacle formé dans ledit moyen de support de l'article pour fixer ledit agent catalytique dans une position sensiblement entre une paire de lentilles de contact ainsi supportées, de telle sorte que cela évite tout contact physique de l'utilisateur avec ledit agent catalytique lors de son utilisation.

3. Appareil selon la revendication 2, dans lequel ledit moyen de fixation comprend, en outre, un élément porteur présentant une partie de type ossature, élargie configurée pour se mettre en prise et supporter ledit agent catalytique et en vue de son insertion amovible dans ledit moyen formant réceptacle.

4. Appareil selon la revendication 3, dans lequel ledit élément porteur comprend des éléments de mise en prise de type à serrage, élastiques, et en ce que ledit moyen de support de l'article comprend des moyens formant projections dans ledit moyen formant réceptacle pour assurer une mise en prise correspondante, de manière amovible, avec lesdits éléments de mise en prise dans ce dernier, de manière à éviter sensiblement tout contact manuel avec l'agent catalytique lors de l'insertion et du retrait par l'utilisateur de l'élément porteur par rapport audit moyen formant réceptacle.

5. Appareil selon la revendication 1 dans lequel ledit moyen de fixation comprend une partie débordante sur ledit moyen de support de l'article, dont la forme est complémentaire à celle dudit agent catalytique pour se mettre en prise, de manière amovible, avec celui-ci.

6. Appareil selon la revendication 1, dans lequel ledit moyen de support de l'article comprend un élément de base formant support de lentille et un élément formant couvercle couplés, de manière articulée, pour entourer en l'enveloppant une lentille par rapport à ladite base de réception de la lentille, et un moyen d'arrêt formé sur ledit élément formant couvercle et ladite base respectivement, pour définir des positions d'arrêt dudit couvercle, dans des positions respectives totalement ouverte et totalement fermée de ce dernier.

7. Appareil selon la revendication 2, dans lequel lesdites surfaces de support des lentilles définissent une structure de type panier présentant une pluralité d'ouvertures traversantes pour laisser passer ladite solution en vue de son contact avec une lentille de contact ainsi supportée, et du nettoyage de cette dernière.

8. Appareil selon la revendication 7 dans lequel ledit moyen de support d'article comprend, en outre, un élément de recouvrement sus-jacent de type panier et dont la forme est complémentaire à celle de ladite surface de support de lentille pour recouvrir et envelopper, une lentille de contact supportée sur ladite surface, ledit élément de recouvrement de type panier présentant également une pluralité d'ouvertures traversantes pour assurer le passage de ladite solution à travers ces dernières, et ledit moyen de support d'article définissant des ouvertures traversantes pour placer ledit moyen formant réceptacle en communication avec ledit récipient adjacent audit moyen formant couvercle de ce dernière pour assurer le libre écoulement de ladite solution et de l'oxygène libéré lors de la décomposition de cette dernière, et à travers ledit moyen de support de l'article pour favoriser le contact étroit de la solution avec lesdites lentilles de contact et assurer le nettoyage de ces dernières.

9. Appareil prévu pour être utilisé avec un agent catalytique lors de la stérilisation de lentilles de contact ou similaire, dans lequel lesdites lentilles sont placées dans une solution de stérilisation contenue dans une cuve de réaction, laquelle solution de stérilisation peut se décomposer lors de son contact avec ledit agent catalytique, ledit appareil comprenant: une cuve de réaction pouvant contenir une solution de stérilisation et comprenant une partie de récipient, un moyen formant couvercle et un moyen formant support d'article comprenant des surfaces de support d'article pour supporter lesdites lentilles de contact en contact avec la solution de stérilisation dans ledit récipient; et comprenant, en outre, des moyens pour fixer ledit agent catalytique sur ledit moyen de support d'article de telle sorte que ledit agent catalytique peut être retiré en vue de son remplacement par un utilisateur lorsque l'agent catalytique est épuisé; ledit moyen de fixation comprenant un moyen formant réceptacle formé dans ledit moyen formant support de l'article pour fixer ledit agent catalytique dans une position sensiblement entre lesdites surfaces de support d'article, de telle sorte que cela évite tout contact physique de l'utilisateur avec ledit agent catalytique lors de l'utilisation; ladite fixation dudit agent catalytique dans ledit moyen formant réceptacle évitant ainsi tout contact de l'agent catalytique avec la solution dans ladite cuve de réaction jusqu'à ce que le moyen de support de l'article à l'intérieur duquel sont placées les lentilles de contact soit placé dans la solution dans le récipient pour commencer le processus de stérilisation, pour éviter ainsi toute décomposition prématurée de la solution de stérilisation avant l'introduction des lentilles de contact en vue de leur stérilisation.

10. Appareil selon la revendication 9, dans lequel ledit moyen de fixation comprend, en outre, un élément porteur présentant une partie de type ossature, élargie, configurée pour se mettre en prise et supporter ledit agent catalytique, et en vue de son insertion amovible dans ledit moyen formant réceptacle.

11. Appareil selon la revendication 10, dans lequel ledit élément porteur comprend, en outre, des éléments de mise en prise de type à serrage, élastiques, et selon lequel ledit moyen de support de l'article comprend des moyens formant projections correspondantes dans ledit moyen formant réceptacle pour assurer une mise en prise amovible avec lesdits éléments de mise en prise dans ces derniers, de manière à éviter sensiblement tout contact manuel avec l'agent catalytique pendant l'insertion et le retrait, par l'utilisateur, de l'élément porteur par rapport audit moyen formant réceptacle.

12. Appareil selon la revendication 9, dans lequel ledit moyen formant support de l'article comprend un élément de base formant support de lentille et un élément formant couvercle couplés, de manière articulée, pour entourer, en l'enveloppant, une lentille par rapport à ladite base de réception de la lentille, et un moyen d'arrêt formé sur ledit élément formant couvercle et ladite base, respectivement, pour définir les positions d'arrêt dudit couvercle dans des positions respectives, totalement ouverte et totalement fermée de ce dernier.

13. Appareil selon la revendication 9, dans lequel lesdites surfaces de support de lentille définissent une structure de type panier présentant une pluralité d'ouvertures traversantes pour laisser passer ladite solution en vue de son contact avec une lentille de contact ainsi supportée et du nettoyage de ces dernières.

14. Appareil selon la revendication 13, dans lequel ledit moyen de support d'article comprend, en outre, un élément de recouvrement sus-jacent, de type panier, dont la forme est complémentaire à celle de ladite surface de support de lentille pour recouvrir et envelopper, une lentille de contact supportée sur ladite surface, ledit élément de recouvrement de type panier présentant également une pluralité d'ouvertures traversantes pour assurer le passage de la solution à travers ces dernières, et ledit moyen de support d'article définissant des ouvertures traversantes pour placer ledit moyen formant réceptacle en communication avec ledit récipient adjacent audit moyen formant couvercle de ce dernier pour assurer l'écoulement libre de ladite solution et de l'oxygène libéré lors de la décomposition de cette dernière autour et à travers dudit moyen de support de l'article pour favoriser le contact étroit de la solution avec lesdites lentilles de contact pour nettoyer ces dernières.

15. Appareil prévu pour être utilisé avec un agent catalytique lors de la stérilisation de lentilles de contact ou similaire, dans lequel lesdites lentilles sont placées dans une solution de stérilisation contenue dans une cuve de réaction, laquelle solution de stérilisation peut se décomposer lors de son contact avec ledit agent catalytique, ledit appareil comprenant: une cuve de réaction pouvant contenir une solution de stérilisation et comprenant une partie de récipient, un moyen formant couvercle et un moyen formant support d'article comprenant des surfaces de support d'article pour supporter lesdites lentilles de contact en contact avec la solution de stérilisation dans ledit récipient; et comprenant, en outre, une partie débordante sur ledit moyen de support de l'article, dont la forme est complémentaire à celle dudit agent catalytique pour se mettre en prise, de manière amovible, avec cette dernière, pour fixer ledit agent catalytique sur ledit moyen de support de telle sorte que ledit agent catalytique peut être retiré pour être remplacé par un utilisateur lorsque l'agent catalytique est épuisé; ladite fixation dudit agent catalytique sur ledit moyen de support de l'article évitant ainsi tout contact de l'agent catalytique avec la solution dans ledit récipient jusqu'à ce que le moyen de support de l'article dans lequel sont placées les lentilles de contact soit placé dans la solution dans la cuve de réaction pour commencer le processus de stérilisation, pour éviter ainsi toute décomposition prématurée de la solution de stérilisation avant l'introduction des lentilles de contact en vue de leur stérilisation dans cette dernière.

16. Appareil prévu pour être utilisé lors de la stérilisation de lentilles de contact ou similaires, dans lequel lesdites lentilles sont placées dans une solution de stérilisation contenue dans une cuve de réaction, laquelle solution de stérilisation peut se décomposer par contact avec un agent catalytique, ledit appareil comprenant: une cuve de réaction pouvant contenir une solution de stérilisation et caractérisé en ce qu'il comprend une partie de récipient, un moyen formant couvercle et un moyen de support d'article pour supporter une lentille de contact en contact avec la solution de stérilisation dans ledit récipient; un agent catalytique contenant un corps recevant le catalyseur de forme prédéterminée revêtu d'une quantité d'un matériau formant catalyseur; et comprenant, en outre, des moyens pour fixer ledit agent catalytique sur ledit moyen de support de l'article de telle sorte que ledit agent catalytique peut être retiré en vue de son remplacement par un utilisateur lorsque l'agent catalytique s'épuise lors de son utilisation; ladite fixation dudit agent catalytique sur ledit moyen de support de l'article évitant ainsi tout contact de l'agent catalytique avec la solution dans ladite cuve de réaction jusqu'à ce que le moyen de support de l'article sur lequel est fixé l'agent catalytique soit placé dans la solution dans le récipient pour commencer le processus de stérilisation, pour éviter toute décomposition prématurée de la solution de stérilisation avant l'introduction des lentilles de contact en vue de leur stérilisation dans cette dernière.

17. Appareil selon la revendication 16, dans lequel ledit moyen de support de l'article comprend des surfaces de support de lentilles pour supporter une paire de lentilles de contact et dans lequel ledit moyen de fixation comprend un moyen formant réceptacle formé dans ledit moyen de support d'article pour fixer ledit agent catalytique dans une position sensiblement entre lesdites surfaces de support des lentilles, de telle sorte que cela permet d'éviter tout contact physique de l'utilisateur et des lentilles avec ledit agent catalytique pendant l'utilisation.

18. Appareil selon la revendication 17, dans lequel ledit moyen de fixation comprend, en outre, un élément porteur comportant une partie formant corps, de type ossature, élargie, et selon lequel le corps de réception du catalyseur est formé de manière à se mettre en prise avec et être supporté par ladite partie formant corps de type ossature.

19. Appareil selon la revendication 18, dans lequel ledit élément porteur comprend, en outre, des éléments de mise en prise de type à serrage, élastiques, et selon lequel ledit moyen de support de l'article comprend des moyens formant projections dans ledit moyen formant réceptacle afin d'assurer une mise en prise correspondante, de manière amovible, avec lesdits éléments de mise en prise dans ce dernier.

20. Appareil selon la revendication 16, dans lequel ledit moyen de fixation comprend une partie débordante sur ledit élément porteur, dont la forme est complémentaire à celle dudit corps de réception du catalyseur pour se mettre en prise, de manière amovible, avec ce dernier.

21. Appareil selon la revendication 6, dans lequel ledit moyen d'arrêt comprend un élément en saillie pouvant fléchir de manière élastique, formé dans ledit élément de base à une position en regard et alignée par rapport à une partie de bord formant couvercle, et au moins une projection formée dans ledit élément formant couvercle qui se met en prise avec l'élément en saillie élastique pendant l'ouverture et la fermeture du couvercle pour définir une position d'arrêt du couvercle vers chaque côté de la zone sur laquelle l'élément en saillie élastique et la projection se mettent en prise lors de l'ouverture et de la fermeture du couvercle.

22. Appareil prévu pour être utilisé lors de la stérilisation de lentilles de contact ou similaires, dans lequel lesdites lentilles sont placées dans une solution de stérilisation contenue dans une cuve de réaction, laquelle solution de stérilisation peut se décomposer par contact avec un agent catalytique, ledit appareil comprenant: un moyen de support d'article pour supporter une ou plusieurs lentilles de contact; un moyen de fixation pour fixer un agent catalytique sur ledit moyen de support d'article de telle sorte que ledit agent catalytique peut être retiré en vue de son remplacement par un utilisateur lors de l'épuisement de l'agent catalytique lors de son utilisation; ledit moyen de fixation comprenant une partie de corps et des moyens de préhension élastiques couplés à ladite partie de corps et des moyens de fixation coopérants formés sur ledit moyen de support d'article pour se mettre en prise avec lesdits moyens de préhension élastiques pour fixer, de manière amovible, ledit moyen de fixation par rapport audit moyen de support d'article; ladite partie de corps étant revêtue d'une quantité de matériau formant catalyseur; ladite fixation dudit agent catalytique sur ledit moyen de support d'article évitant ainsi tout contact de l'agent catalytique avec la solution dans ledit récipient jusqu'à ce que le moyen de support d'article sur lequel est fixé l'agent catalytique soit placé dans la solution dans la cuve de réaction pour commencer le processus de stérilisation, pour éviter ainsi toute décomposition prématurée de la solution de stérilisation avant l'introduction des lentilles de contact en vue de leur stérilisation dans cette dernière.

23. Appareil catalyseur prévu pour être utilisé lors de la stérilisation de lentilles de contact ou similaires, dans lequel lesdites lentilles sont placées dans un moyen de support d'article dans une solution de stérilisation contenue dans une cuve de réaction, laquelle solution de stérilisation peut se décomposer par contact avec un agent catalytique, ledit appareil catalyseur comprenant: un moyen de fixation pour fixer un agent catalytique sur ledit moyen de support d'article de telle sorte que ledit agent catalytique peut être retiré en vue de son remplacement par un utilisateur, lorsque l'agent catalytique est épuisé lors de son utilisation, ledit moyen de fixation comprenant une partie de corps et un moyen de serrage à encliquetage couplé à ladite partie de corps et configuré pour se mettre en prise avec un moyen de fixation coopérant formé sur ledit moyen de support d'article en vue d'assurer une fixation amovible par rapport audit moyen de support d'article; ladite partie de corps étant revêtue d'une quantité de matériau formant catalyseur et un outil d'insertion/retrait comportant des moyens de mise en prise formés pour se mettre en prise avec, et se dégager de ladite partie de corps en vue de sa fixation par rapport audit moyen de support d'article et pour se mettre en prise avec ladite partie de corps en vue de son retrait dudit moyen de support d'article; ladite fixation dudit agent catalytique sur ledit moyen de support de l'article évitant ainsi tout contact de l'agent catalytique avec la solution dans ladite cuve de réaction jusqu'à ce que le moyen de support de l'article sur lequel est fixé l'agent catalytique soit placé dans la solution dans le récipient pour commencer le processus de stérilisation, pour éviter toute décomposition prématurée de la solution de stérilisation avant l'introduction des lentilles de contact en vue de leur stérilisation dans cette dernière.

24. Appareil catalyseur prévu pour être utilisé lors de la stérilisation de lentilles de contact ou similaires, dans lequel lesdites lentilles sont placées dans un moyen de support d'article dans une solution de stérilisation contenue dans une cuve de réaction, laquelle solution de stérilisation peut se décomposer par contact avec un agent catalytique, ledit appareil catalyseur comprenant:un agent catalytique comprenant un corps de réception de catalyseur de forme prédéterminée revêtue d'une quantité d'un matériau formant catalyseur; et comprenant, en outre, des moyens pour fixer ledit agent catalytique sur ledit moyen de support d'article de telle sorte que ledit agent catalytique peut être retiré en vue de son remplacement par un utilisateur lors de l'épuisement de l'agent catalytique lors de son utilisation; ledit moyen de fixation comprenant un élément porteur présentant une partie de corps en forme de ossature, élargie, et selon lequel ledit corps de réception du catalyseur est formé pour se mettre en prise avec et être supporté par ladite partie de corps de type ossature; ladite fixation dudit agent catalytique sur ledit moyen de support de l'article évitant ainsi tout contact de l'agent catalytique avec la solution dans ledit récipient jusqu'à ce que le moyen de support d'article auquel est fixé le moyen de support d'article soit placé dans la solution dans le récipient pour commencer le processus de stérilisation, et pour éviter ainsi toute décomposition prématurée de la solution de stérilisation avec l'introduction des lentilles de contact en vue de leur stérilisation dans cette dernière.

25. Appareil selon la revendication 24, dans lequel ledit élément porteur comprend, en outre, des éléments de mise en prise de type à serrage, élastiques, placés et configurés en vue de leur mise en prise correspondante, de manière amovible, avec ledit moyen de support d'article.

26. Appareil porteur de lentilles, prévu pour être utilisé avec un agent catalytique lors de la stérilisation de lentilles de contact ou similaires, dans lequel lesdites lentilles sont placées dans une solution de stérilisation contenue dans une cuve de réaction, laquelle solution de stérilisation peut se décomposer par contact avec ledit agent catalytique, ledit appareil porteur de lentilles comprenant: une structure de support d'article pour supporter une lentille de contact en contact avec la solution de stérilisation dans ladite cuve de réaction; ladite structure de support d'article comprenant un élément formant base de support de lentille et un élément formant couvercle couplés, de manière articulée, audit élément formant base pour entourer, en l'enveloppant, une lentille par rapport au dit élément formant base de réception de lentille, et un moyen d'arrêt formé sur ledit élément formant couvercle et ledit élément formant base respectivement pour définir des positions d'arrêt dudit élément formant couvercle dans des positions respectives totalement ouverte et fermée; selon lequel ledit moyen d'arrêt comprend un élément en saillie pouvant être fléchi de manière élastique formé dans ledit élément formant base à une position située en regard et alignée par rapport au moins une partie de l'élément formant couvercle, et au moins une projection formée dans ledit élément formant couvercle qui se met en prise avec l'élément en saillie élastique pendant l'ouverture et la fermeture de l'élément formant couvercle pour définir une position d'arrêt de l'élément formant couvercle vers chaque côté de la zone avec laquelle ledit élément en saillie élastique et la projection se mettent en prise lors de l'ouverture et de la fermeture de l'élément formant couvercle.
